**Europäisches Patentamt**

**European Patent Office**　　⑪　Veröffentlichungsnummer: **0 035 597**
**B1**
**Office européen des brevets**

⑫　　　　**EUROPÄISCHE PATENTSCHRIFT**

④⑤　Veröffentlichungstag der Patentschrift:　　�localhost　Int. Cl.³: **A 61 K 31/135, C 07 C 87/453**
**01.08.84**

㉑　Anmeldenummer: **80107848.6**

㉒　Anmeldetag: **12.12.80**

⑤④　**Pharmazeutische Zubereitungen.**

(30)　Priorität: **08.03.80 DE 3008993**

④③　Veröffentlichungstag der Anmeldung:
**16.09.81 Patentblatt 81/37**

④⑤　Bekanntmachung des Hinweises auf die Patenterteilung:
**01.08.84 Patentblatt 84/31**

㉘④　Benannte Vertragsstaaten:
**AT CH DE FR GB IT LI**

㉟⑥　Entgegenhaltungen:
**CHEMICAL ABSTRACTS, Band 85, Nr. 7, 16. August
1976, Seite 31, Nr. 40768t Columbus, Ohio, U.S.A. J.A.
FUENTES et al.: "Comparison of the apparent
antidepressant activity of (-)- and (+)-tranylcypromine in
an animal model"
JOURNAL OF MEDICINAL AND PHARMACEUTICAL
CHEMISTRY, Band 5, November 1962, Seiten 1265-1284
C.L. ZIRKLE et al.: "2-Substituted cyclopropylamines. II.
Effect of structure upon monoamine oxidase-inhibitory
activity as measured in vivo by potentiation of tryptamine
convulsions"**

Die Akte enthält technische Angaben, die nach dem
Eingang der Anmeldung eingereicht wurden und die
nicht in dieser Patentschrift enthalten sind.

㉃③　Patentinhaber: **Röhm Pharma GmbH,
Dr.-Otto-Röhm-Strasse 2-4, D-6108 Weiterstadt 1 (DE)**

㉃②　Erfinder: **Völger, Karl-Dieter, Dr., Bebelstrasse 10,
D-6101 Bickenbach (DE)**
Erfinder: **Treudler, Ilse, Sudetenstrasse 18,
D-6101 Messel (DE)**

## Beschreibung

Die therapeutische Verwendung des (–)-Trans-2-Phenylcyclopropylamins zum zeitweiligen Abschwächen von Depressionssymptomen ohne wesentliche Nebenwirkungen beim Menschen und eine entsprechende Zusammensetzung ist aus der DE-OS 25 12 893 bekannt. Es wird darin die Forderung betont, die Zusammensetzung müsse im wesentlichen frei von (+)-Trans-2-Phenylcyclopropylamin sein.

Aus der US-PS 2 997 422 war bekannt gewesen, dass das Trans-Isomere des 2-Phenylcyclopropylamins (Tranylcypromin) als Inhibitor des körpereigenen Enzyms Monoaminoxidase wirkt.

Es war ferner festgestellt worden, dass das (+)-Enantiomere des Tranylcypromins eine 15–20mal höhere Monoaminoxidase-Wirkung besitzt als das (–)-Enantiomere, aber andererseits das (–)-Enantiomere ein um ein Mehrfaches besserer Hemmer der Catecholamin-Aufnahme in die Hirn-Synaptosomen darstellt als das (+)-Enantiomere.

Da nun ein klinischer Vergleich die eindeutige Überlegenheit des (–)-Enantiomeren des Tranylcypromins als Antidepressivum gekoppelt mit geringeren Nebenwirkungen, verglichen mit dem (+)-Enantiomeren ergab, drängt sich die Vorstellung auf, die antidepressive Wirkung beruhe auf dem Hemmeffekt gegenüber der Catecholamin-Aufnahme in die Hirn-Synaptosomen. [Vgl. S. Snyder et al. in J. Pharmac. Exp. Therapeutics 180, (3) (1972), S. Snyder et al. in «L-Dopa Behaviour», pg. 35–56 Ed. Malitz, Raven Press 1972, S.H. Snyder, J. Psychiatr. Res. 10, 153 (1974)].

Von daher gesehen ist es nur folgerichtig, die Anwendung des (+)-Trans-2-phenylcyclopropylamins aus der Therapie auszuschliessen, wie dies in der eingangs genannten DE-OS 25 12 893 geschehen ist.

Es wurde nun gefunden, dass sich das Parkinson-Syndrom (Morbus Parkinson) mit sehr gutem Erfolg therapeutisch behandeln lässt, wenn man (+)-Trans-2-phenylcyclopropylamin oder ein physiologisch unbedenkliches Säureadditionssalz desselben als Wirkstoff verwendet.

Das Parkinson-Syndrom weist eine Vielzahl von Symptomen auf, die sich in 3 Gruppen unterteilen lassen.

(1) motorische Störungen [mit Plus-Symptomen, wie Tonussteigerung der quergestreiften Muskulator (Rigor) sowie Tremor und Minus-Symptomen, wie Verminderung der Gesamtmotorik (Akinese) und Verlust der Stellreflexe].

(2) Vegetative Symptome (vermehrter Speichel- und Tränenfluss) und

(3) psychische Störungen (erschwerte Entschlussfähigkeit, depressive Verstimmung u.a.).

Für das meist zwischen dem 40.–60. Lebensjahr beginnenden Leiden wird der Untergang von Nervenzellen in den motorischen Kerngebieten des Hirnstamms auf degenerativer Basis verantwortlich gemacht.

Eine rationelle Herstellung des Trans-2-Phenylcyclopropylamins ist beispielsweise in der US-PS 4 016 204 beschrieben. Die Auftrennung in das (+)- bzw. das (–)-Enantiomere des Tranylcypramins ist beispielsweise von Kaiser im Journal of Medicinal and Pharmaceutical Chemistry 5, 1243 (1962) beschrieben worden.

Das von der Herstellung des Trans-2-Phenylcyclopropylamins her vorliegende Racemat kann nach den klassischen Verfahren der Racematspaltung optisch aktiver Amine, d.h. durch Salzbildung mit optisch aktiven Säuren, wie z.B. der Weinsäure, aufgearbeitet werden.(Vgl. Houben-Weyl, Band IV, Teil II, S. 513–519, Georg Thierne Verlag 1955).

Als physiologisch bzw. pharmazeutisch unbedenkliche Säureadditionssalze des (+)-Tranylcypramins können erfindungsgemäss Salze von anorganischen Säuren, wie z.B. der Schwefel-, Salpeter-, Phosphor- und Salzsäure, aber auch die von organischen Säuren wie der Essig-, Propion-, Bernstein-, Fumar-, Malein-, Zitronen-, Wein-, Zimt-, p-Aminobenzoe-, p-Acetaminobenzoesäure, Salicyl-, Acetylsalicyl-, Milch-, Mandel-, Äthandisulfonsäure u.ä. verwendet werden. Die Herstellung der Säureadditionssalze kann in an sich bekannter Weise, beispielsweise durch Lösen des freien (+)-Tranylcypramins in einem geeigneten Lösungsmittel und Zugabe der gewünschten Säure, beispielsweise in stöchiometrischem Verhältnis und Isolierung des gebildeten Säureadditionssalzes geschehen.

Als Lösungsmittel kommen neben Wasser beispielsweise Alkohole, wie Äthanol, Methanol, Ketone wie Aceton, Methylisopropylketon, Äther wie Diäthyläther, Dioxan, Ester wie Essigester, (wässrige) Säuren wie Essigsäure, Salzsäure, Schwefelsäure sowie Kombinationen von Lösungsmitteln in Frage. Die erfindungsgemässen Zubereitungen können pharmazeutisch unbedenkliche Träger- und Hilfsstoffe enthalten; sie können ferner mit anderen Wirkstoffen kombiniert werden. Streng zu beachten sind jedoch die von Monoaminoxidasehemmern (Thymeretika) her bekannten Kautelen. Insbesondere ist die Verabreichung und der Konsum von z.B. in der Nahrung enthaltenen Aminen sorgfältig zu kontrollieren.

Die gleichzeitige Gabe von sympathomimetischen Aminen ist zu vermeiden, ebenso stellen Nahrungsmittel, die grössere Mengen an blutdrucksteigernden biogenen Aminen enthalten, wie z.B. Käse, eine potentielle Gefährdung dar.

Die erfindungsgemässen pharmazeutischen Zubereitungen können parenteral und enteral zur Anwendung gelangen; sie lassen sich auf die übliche Weise unter Verwendung der geläufigen Träger- und Hilfsstoffe bzw. Lösungsmittel herstellen. Bevorzugt ist die orale Verabreichungsform. Eine Ausführungsform der Erfindung stellen feste, zur oralen Verabreichung geeignete Zubereitungen dar, wie z.B. Tabletten, Kapseln, Dragees usw. Für die orale Applikation können als Trägermaterialien pharmazeutisch indifferente Feststoffe, wie beispielsweise Mannit, Milch-

zucker, organische oder anorganische Calcium-salze etc. verwendet werden. Dabei kann die verwendete Menge an festem Träger weitgehend variiert werden. Zweckmässig ist beispielsweise die Verwendung von ca. 25 mg bis 1 g an festem Träger.

Als Bindemittel kommen u.a. Polyvinylpyrrolidon, Gelatine oder Cellulosederivate in Frage. Als weitere Zusätze können Tablettensprengmittel, wie beispielsweise Stärke oder Alginsäure, Gleitmittel, wie z.B. Stearinsäure oder deren Salze und anorganische Fliessmittel, wie z.B. Talk oder kolloidale Kieselsäure, sowie Geschmackskorrigentien, verwendet werden.

Der Wirkstoff (+)-Tranylcypramin bzw. seine Säureadditionssalze können mit den Hilfsstoffen in üblicher Weise gemischt und nass oder trocken granuliert werden. Je nach Art der verwendeten Zusatzstoffe kann gegebenenfalls auch durch einfaches Mischen ein direkt tablettierbares Pulver erhalten werden. Das Granulat oder Pulver kann direkt in Kapseln abgefüllt oder in üblicher Weise zu Tablettenkernen verpresst werden.

Auch die Verabreichung mittels Ampullen und Suppositorien kommt in Frage. Die Dosierungen bei den erfindungsgemässen pharmazeutischen Präparaten richten sich nach Art und Schwere der Erkrankung, Alter und Disposition des Patienten, sowie den üblicherweise zu berücksichtigenden individuellen Faktoren. Die vorzugsweise verabreichte Dosierung liegt im Bereich von 1–100 mg/Tag, besonders bevorzugt 1–20 mg/Tag, speziell 1–10 mg/Tag, an dem Wirkstoff (+)-Tranylcypromin bzw. diesen Mengen entsprechenden Quantitäten der Säureadditionssalze bei der Therapie des Parkinsonsyndroms. Die Verabreichung kann – in Abhängigkeit von der als adäquat befundenen galenischen Formulierung – ein- bis etwa viermal pro Tag erfolgen.

Das folgende Beispiel dient zur Erläuterung der erfindungsgemässen pharmazeutischen Zubereitung.

Beispiel:
Herstellungsvorschrift für (+)-Tranyl-Cyprominsulfat-Kapseln

a) Aufbereitung des Wirkstoffs
Der Wirkstoff wird auf einer Mühle gemahlen.
b) Kapselfüllmasse/Rezeptur
(+)-Trans-2-phenyl-cyclopropylamin-sulfat

| | |
|---|---:|
| | 4,04 g |
| Magnesiumstearat | 0,50 g |
| Milchzucker DAB | 95,46 g |
| | 100,00 g |

Alle Bestandteile sind mit einem Zwangsmischer zu mischen. Die Mischung wird über ein Sieb von 0,06 mm Maschenweite gesiebt.
c) Kapselherstellung
Die vorstehende Mischung wird auf einer Kapselfüllmaschine in Hartgelatine-Kapseln, Grösse 4, verfüllt. Das Füllgewicht beträgt 170 mg. Eine Kapsel enthält 6,87 mg Wirkstoff.

## Patentansprüche

1. Pharmazeutische Zubereitungen zur medikamentösen Therapie des Parkinson-Syndroms, gekennzeichnet durch einen Gehalt einer wirksamen Menge an (+)-Trans-2-phenylcyclopropylamin, das im wesentlichen von (−)-Trans-2-phenylcyclopropylamin frei ist, und/oder eines physiologisch unbedenklichen Säureadditionssalzes als Wirkstoff.

2. Pharmazeutische Zubereitungen zur medikamentösen Therapie des Parkinson-Syndroms, gemäss Anspruch 1, dadurch gekennzeichnet, dass sie pro Verabreichungseinheit 1–100 mg, vorzugsweise 1–20 mg, (+)-Trans-2-phenylcyclopropylamin und/oder die entsprechende Menge eines physiologisch unbedenklichen Säureadditionssalzes desselben enthalten.

3. Verwendung des im wesentlichen vom optischen Enantiomeren freien (+)-Trans-2-phenylcyclopropylamins und/oder seiner physiologisch unbedenklichen Säureadditionssalze zur Herstellung therapeutischer Zubereitungen zur medikamentösen Therapie des Parkinsonismus.

4. Pharmazeutische Zubereitungen zur medikamentösen Therapie des Parkinson-Syndroms, dadurch gekennzeichnet, dass sie pro Verabreichungseinheit 1–10 mg (+)-Trans-2-phenylcyclopropylamin und/oder die entsprechende Menge eines physiologisch unbedenklichen Säureadditionssalzes desselben enthalten.

## Claims

1. Pharmaceutical preparations for medicamentary therapy of Parkinson's disease, characterised in that they contain an effective quantity of (+)-trans-2-phenylcyclopropylamine, which is substantially free from (−)-trans-2-phenylcyclopropylamine, and/or a physiologically harmless acid addition salt as active substance.

2. Pharmaceutical preparations for medicamentary therapy of Parkinson's disease, as claimed in claim 1, characterised in that they contain, per dosage unit, 1–100 mg, preferably 1–20 mg, of (+)-trans-2-phenylcyclopropylamine and/or the corresponding quantity of a physiologically harmless acid addition salt thereof.

3. Use of (+)-trans-2-phenylcyclopropylamine, substantially free from the optical enantiomer, and/or of the physiologically harmless acid addition salts thereof for the production of therapeutical preparations for medicamentary therapy of Parkinsonism.

4. Pharmaceutical preparations for medicamentary therapy of Parkinson's disease, characterised in that they contain, per dosage unit, 1–10 mg of (+)-trans-2-phenylcyclopropylamine and/or the corresponding quantity of a physiologically harmless acid addition salt thereof.

## Revendications

1. Préparations pharmaceutiques pour le traitement médicamenteux du syndrome de Parkinson, caractérisé en ce qu'elles contiennent une quantité active de 2-phénylcyclopropylamine trans-(+) qui est pratiquement exempte de 2-phénylcyclopropylamine trans-(-), et/ou d'un sel formé par addition d'un acide physiologiquement acceptable, en tant que substance active.

2. Préparations pharmaceutiques pour le traitement médicamenteux du syndrome de Parkinson selon la revendication 1, caractérisées en ce qu'elles contiennent, par dose unitaire, 1 à 100 mg, de préférence 1 à 20 mg de 2-phénylcyclopropylamine trans-(+) et/ou la quantité correspondante d'un sel de celle-ci, formé par addition d'un acide et acceptable physiologiquement.

3. Utilisation de la 2-phénylcyclopropylamine trans-(+) pratiquement exempte d'énantiomère optique et/ou de ses sels formés par addition d'acides et acceptables physiologiquement, pour la fabrication de préparations thérapeutiques pour le traitement médicamenteux du parkinsonisme.

4. Préparations pharmaceutiques pour le traitement médicamenteux du syndrome de Parkinson, caractérisées en ce qu'elles contiennent, par dose unitaire, 1 à 10 mg de 2-phénylcyclopropylamine trans-(+) et/ou la quantité correspondante d'un sel de celle-ci, formé par addition d'un acide et acceptable physiologiquement.